# EUROPEAN PATENT SPECIFICATION

(11) **EP 4 289 473 B1**
(45) Date of publication and mention of the grant of the patent: **11.11.2026**
(21) Application number: 21924879.6
(22) Date of filing: 24.12.2021
(51) Int. Cl.: A61N 5/06

(54) **LIGHT IRRADIATION HAIR REMOVAL DEVICE**
LICHTBESTRAHLUNGSVORRICHTUNG ZUR HAARENTFERNUNG
DISPOSITIF D'ÉPILATION PAR IRRADIATION LUMINEUSE

(30) Priority: 05.02.2021 JP 2021017232
(43) Date of publication of application: 13.12.2023
(73) Proprietor: Panasonic Intellectual Property Management Co., Ltd., Kadoma-shi, Osaka 571-0057 (JP)
(72) Inventor: SAKAMOTO, Ryohei, Kadoma-shi, Osaka 571-0057 (JP); KASUGAI, Hideki, Kadoma-shi, Osaka 571-0057 (JP)
(74) Representative: Grünecker Patent- und Rechtsanwälte PartG mbB
(86) International application number: PCT/JP2021/048151
(87) International publication number: WO 2022/168494

(56) References cited:
- CN-A- 106 964 067
- GB-A- 2 410 690
- JP-A- 2002 010 825
- JP-A- 2003 265 498
- JP-B2- 5 715 128
- US-A1- 2004 092 917
- US-A1- 2007 255 355
- US-A1- 2009 093 799
- US-A1- 2010 204 762
- US-A1- 2012 116 371
- US-A1- 2012 172 949
- US-A1- 2014 121 631
- US-A1- 2019 374 791
- US-B1- 6 595 985

## Description

The present disclosure relates to a light irradiation hair removal device.

In recent years, as a means for hair removal of human body hair, a method is known, in which a hair removal effect is obtained by irradiating a skin surface with light of a predetermined wavelength to generate heat and damage cells near hair follicles. Examples of such a device include a light irradiation hair removal device.

The light irradiation hair removal device includes a light emitting unit having a light source. Further, as a light source of the light irradiation hair removal device, a light source having a narrowband wavelength spectrum or a light source having a broadband wavelength spectrum is generally used (see, for example, PTL 1 and PTL 2). Examples of the light source having a narrowband wavelength spectrum, include light emitting diodes (LED), laser, or the like. Examples of the light source having a broadband wavelength spectrum include a flash lamp.

PTL 1 describes among others a light irradiation hair removal device that emits a processing light pulse in a range from 1 ms to 600 ms by a light source (VCSEL: Vertical Cavity Surface Emitting LASER) having a peak emission wavelength in a wavelength range from 570 nm to 1200 nm. The light irradiation hair removal device described in PTL 1 obtains a hair removal effect by applying irradiation energy in a range from 2 J/cm² to 30 J/cm². PTL 2 describes a light irradiation hair removal device including a light emitting unit having an LED die with a peak emission wavelength in the range of red to near infrared wavelengths from 700 nm to 980 nm. The light irradiation hair removal device described in PTL 2 obtains a hair removal effect by emitting a processing light pulse in a range from 60 ms to 120 ms from a light emitting unit, and applying irradiation energy in a range from 3 J/cm² to 7 J/cm² to the skin.

Each of the light irradiation hair removal devices described in PTL 1 and PTL 2 performs a hair removal treatment by emitting light from a light source for a predetermined time in a state where the light source emitting light of a predetermined wavelength is brought close to a skin surface to be subjected to hair removal. In other words, the light irradiation hair removal devices described in PTL 1 and PTL 2 damage cells near hair follicles by heat generation during light irradiation to obtain a hair removal effect. PTLs 3 to 5 describe further devices that can remove or damage hair.
PTL 1: Japanese Patent No. 5715128
PTL 2: Japanese Translation of PCT International Application No. 2019-509087
PTL 3: US 6,595,985 B1
PTL 4: US 2019/0374791 A1
PTL 5: US 2010/0204762 A1
PTL 6: GB 2 410 690 A
PTL 7: CN 106964067 A
PTL 8: US 2004/0092917 A1
PTL 9: US 2012/0116371 A1
PTL 10: US 2009/0093799 A1
PTL 11: US 2012/0172949 A1

In a conventional light irradiation hair removal device as described above, a good hair removal effect can be obtained when strong light is emitted at a single time. However, since a temperature of the skin suddenly rises when stronger light is emitted to obtain a sufficient hair removal effect, the conventional light irradiation hair removal device may cause discomfort such as damage to a user. In particular, since skin properties and sensitivity to pain of the user are various, there is a trade-off relationship between obtaining a sufficient hair removal effect and suppressing discomfort during light irradiation, and it is difficult to achieve both.

The present disclosure provides a light irradiation hair removal device capable of achieving both a sufficient hair removal effect and suppression of discomfort during light irradiation.

This is achieved by the features of independent claim 1.
Fig. 1 is a cross-sectional view illustrating a configuration of a light irradiation hair removal device according to the present exemplary embodiment.
Fig. 2 is a cross-sectional view taken along line II-II in Fig. 1.
Fig. 3 is a perspective view illustrating an example of a schematic arrangement state of a light source according to the present exemplary embodiment.
Fig. 4 is a block diagram of major parts of the light irradiation hair removal device according to the present exemplary embodiment.
Fig. 5 is a graph illustrating one example of a relationship between elapsed time and light density during light irradiation of the light irradiation hair removal device according to the present exemplary embodiment.
Fig. 6 is a graph illustrating another example of the relationship between elapsed time and light density during light irradiation of the light irradiation hair removal device according to the present exemplary embodiment.

Hereinafter, exemplary embodiments will be described in detail with reference to the drawings. However, detailed descriptions more than necessary may be omitted. For example, detailed descriptions of already well-known matters or redundant descriptions of substantially the same configuration may be omitted.

Note that, the accompanying drawings and the following description are only presented to help those skilled in the art fully understand the present disclosure, and are not intended to limit the subject matters described in the scope of claims.

Further, in the following exemplary embodiment, up-down direction Z of light irradiation hair removal device 1 is defined in a state where an emission port is directed upward. Further, a longitudinal direction in a horizontal direction of light irradiation hair removal device 1 is defined as width direction Y. Furthermore, a short side direction in the horizontal direction of light irradiation hair removal device 1 (a direction orthogonal to up-down direction Z and width direction Y) is defined as front-back direction X.

Further, in the present exemplary embodiment, a side on which a switch unit (not illustrated) is provided in the body of light irradiation hair removal device 1 is defined as a front in the front-back direction.

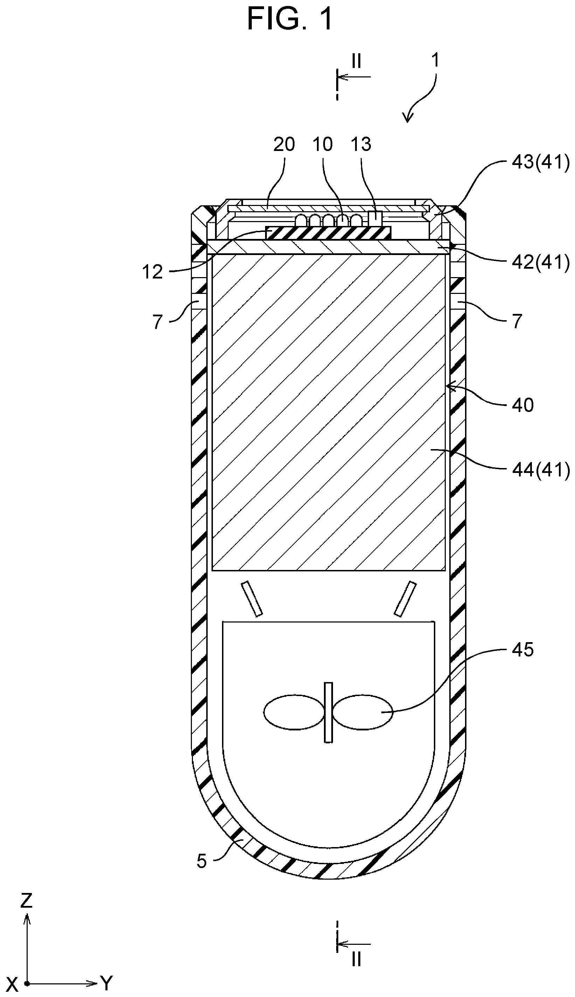

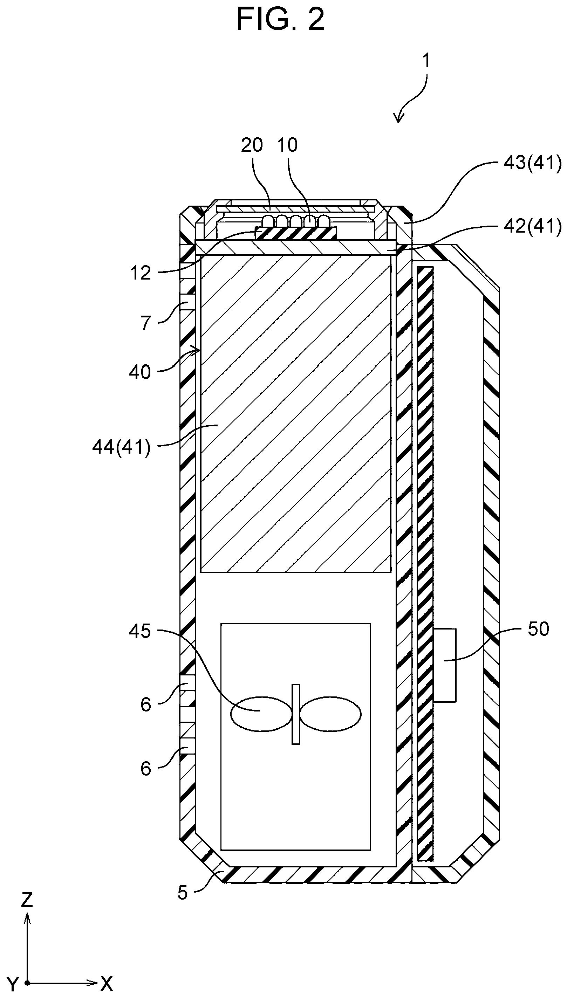

Fig. 1 is a cross-sectional view illustrating a configuration of light irradiation hair removal device 1 according to the present exemplary embodiment, and Fig. 2 is a cross-sectional view taken along a line II-II in Fig. 1. As shown in Figs. 1 and 2, light irradiation hair removal device 1 includes housing 5, light source 10, temperature sensor 13, skin cooling unit 20, cooler 40, and control unit 50.

One end of housing 5 is provided with an opening portion serving as a light emission port of light irradiation hair removal device 1. Light source 10 is provided in the opening portion of housing 5, and emits light on the skin. Further, a bottom portion is formed on a side of housing 5 opposite to light source 10. Housing 5 is provided with a plurality of first opening portions 6 and a plurality of second opening portions 7, and external air is taken in from the plurality of first opening portions 6 and discharged from the plurality of second opening portions 7. Inside housing 5, light source 10, temperature sensor 13, skin cooling unit 20, cooler 40, and control unit 50 are accommodated.

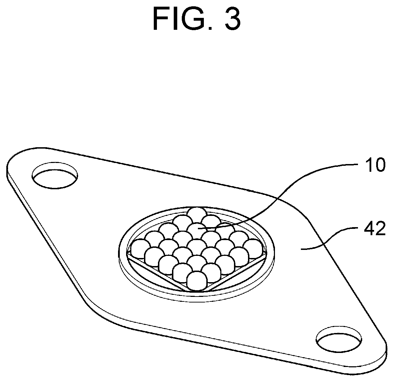

Fig. 3 is a perspective view illustrating an example of a schematic arrangement state of light source 10 according to the present exemplary embodiment. Note that, in Fig. 3, configurations of temperature sensor 13, skin cooling unit 20, and cooler 40 are partially omitted. As shown in Figs. 1 to 3, in the present exemplary embodiment, light source 10 includes a plurality of light emitting diodes (LEDs). The LEDs are mounted on substrate 12 in a state of being spaced at substantially equal intervals. Light source 10 is electrically connected to a power supply (not illustrated), and when power is supplied from the power supply, light is emitted from light source 10.

Light source 10 emits light having a wavelength from 400 nm to 1200 nm inclusive. When the light as described above is emitted on the skin, melanin of hair follicles absorbs the light and generates heat. Then, hair matrixes included in the hair follicles are damaged by the heat, and a hair discharge is promoted. A wavelength of light may be more than or equal to 500 nm, more than or equal to 600 nm, more than or equal to 700 nm, or more than or equal to 800 nm. Further, a wavelength of light may be less than or equal to 1000 nm, or less than or equal to 900 nm. The light emitted from light source 10 may be light having a peak wavelength within a range from 400 nm to 1200 nm inclusive. Even in a case where light has a peak wavelength within the range as described above, the emitted light may contain a wavelength component outside the above range. Further, each of the LEDs does not need to have the same wavelength spectrum, and LEDs that emit light of different wavelength spectra may be used in combination. Note that, the wavelength is a wavelength of light emitted in a case where the temperature of light source 10 is 25°C.

Light source 10 preferably emits light under a condition that an irradiance is more than 15 W/cm² and less than 50 W/cm². By irradiating the hair with light at an irradiance of more than 15 W/cm², it is possible to achieve a good hair removal effect on hair from an early growth period to a growth period. Further, by irradiating the skin with light at an irradiance of less than 50 W/cm², it is possible to suppress a rise in skin temperature due to light irradiation. Therefore, the skin is cooled more reliably, and skin irritation can be reduced. An irradiance may be more than 20 W/cm², more than 25 W/cm², or more than 30 W/cm². Further, the irradiance may be less than 45 W/cm², or less than 40 W/cm².

Skin cooling unit 20 is disposed at a position facing light source 10. Skin cooling unit 20 may be in contact with light source 10, or may be disposed with a space from light source 10. Further, skin cooling unit 20 is provided so as to be in contact with the skin on a side opposite to light source 10. Skin cooling unit 20 is made of a material having translucency. When light source 10 emits light, skin cooling unit 20 transmits light emitted from light source 10, and the skin is irradiated with light transmitted through skin cooling unit 20. Skin cooling unit 20 is, for example, a plate having translucency, and in the present exemplary embodiment, skin cooling unit 20 of a disk is used.

Skin cooling unit 20 is preferably made of a material that does not absorb much of the light emitted from light source 10. Specifically, a total light transmittance of skin cooling unit 20 is preferably more than or equal to 80%. When the total light transmittance is more than or equal to 80%, most of light emitted from light source 10 can be transmitted through skin cooling unit 20. Therefore, a large amount of light can reach melanin, which can promote the hair removal effect. Further, since the amount of light absorbed by skin cooling unit 20 and converted into heat can be reduced, a temperature rise of skin cooling unit 20 can be suppressed. From a viewpoint of making it difficult for skin cooling unit 20 to absorb light, the total light transmittance is more preferably more than or equal to 90%, still more preferably more than or equal to 95%, and particularly preferably more than or equal to 99%. An upper limit value of the total light transmittance is 100%. The total light transmittance can be measured according to JIS K7361-1:1997.

The refractive index of skin cooling unit 20 is preferably 1.7 or higher. When the refractive index of skin cooling unit 20 is more than or equal to 1.7, light from light source 10 is not easily absorbed by skin cooling unit 20. Skin cooling unit 20 becomes easier to transmit light as the refractive index value increases. Therefore, the refractive index is more preferably more than or equal to 1.8, still more preferably more than or equal to 1.9, and particularly preferably more than or equal to 2.0. The upper limit value of the refractive index is not particularly limited, but may be 10. The refractive index can be measured by a minimum deviation method according to JIS B7071-1:2015.

Skin cooling unit 20 cools a skin when it comes into contact with the skin. Skin cooling unit 20 preferably includes a material having a high thermal conductivity. A thermal conductivity of skin cooling unit 20 is preferably more than or equal to 1 W/mK. When the thermal conductivity is more than or equal to 1 W/mK, even when skin cooling unit 20 is heated by light from light source 10 and the skin, heat is easily dissipated, and thus the skin can be effectively cooled. As a value of the thermal conductivity increases, the thermal conductivity of skin cooling unit 20 tends to increase, and a cooling effect of skin cooling unit 20 tends to be better. Therefore, from a viewpoint of cooling efficiency, the thermal conductivity of skin cooling unit 20 is more preferably more than or equal to 2 W/mK, still more preferably more than or equal to 10 W/mK, particularly preferably more than or equal to 30 W/mK, and most preferably more than or equal to 100 W/mK. The upper limit value of the thermal conductivity is not particularly limited, but may be 100,000 W/mK. The thermal conductivity can be measured by a laser flash method according to JIS R1611:2010.

Skin cooling unit 20 may contain an inorganic substance. Specifically, skin cooling unit 20 preferably includes at least one selected from the group consisting of Al₂O₃, ZnO, ZrO₂, MgO, GaN, AlN, and diamond. Since these materials have a high refractive index and a high thermal conductivity, the translucency and thermal conductivity of skin cooling unit 20 can be improved. Note that, Al₂O₃ (sapphire) has a refractive index of 1.79 and a thermal conductivity of 42 W/mK. ZnO has a refractive index of 2.01 and a thermal conductivity of 20 W/mK. ZrO₂ has a refractive index of 2.13 and a thermal conductivity of 3 W/mK. MgO has a refractive index of 1.74 and a thermal conductivity of 47 W/mK. GaN has a refractive index of 2.346 and a thermal conductivity of 200 W/mK. AlN has a refractive index of 2.175 and a thermal conductivity of 150 W/mK. Diamond has a refractive index of 2.417 and a thermal conductivity of 1000 W/mK.

Skin cooling unit 20 may contain resin such as a silicone resin, from the viewpoint of heat resistance and translucency. Further, skin cooling unit 20 may include a resin such as a silicone resin and a highly thermally conductive filler dispersed in the resin. Since skin cooling unit 20 includes the highly thermally conductive filler, heat of skin cooling unit 20 is easily dissipated, and thus the skin can be effectively cooled. The highly thermally conductive filler may contain an inorganic substance as described above.

The proportion of the inorganic substance in skin cooling unit 20 is preferably 10 mass% or higher. By setting the proportion of the inorganic substance in skin cooling unit 20 to more than or equal to 10 mass%, the thermal conductivity of skin cooling unit 20 can be improved. The proportion of the inorganic substance in skin cooling unit 20 is more preferably 30 mass% or higher, still more preferably 50 mass% or higher, particularly preferably 70 mass% or higher, and most preferably 90 mass% or higher.

Skin cooling unit 20 is preferably cooled to be more than or equal to -5°C and less than 35°C. By cooling skin cooling unit 20 to be more than or equal to -5°C, it is possible to cool the skin such that pain in the skin caused by the cooling is unlikely to occur. On the other hand, when skin cooling unit 20 is cooled to be less than 35°C, inflammation due to the rise in skin temperature during light irradiation can be suppressed. Skin cooling unit 20 is more preferably cooled to be more than or equal to 0°C, still more preferably more than or equal to 5°C, and particularly preferably more than or equal to 10°C. Further, skin cooling unit 20 is more preferably cooled to be less than 30°C, still more preferably less than 25°C, particularly preferably less than 20°C, and most preferably less than 15°C.

Temperature sensor 13 detects the temperature of skin cooling unit 20. By detecting the temperature of skin cooling unit 20, the temperature of skin cooling unit 20 can be controlled precisely. Temperature sensor 13 is provided so as to face skin cooling unit 20. Specifically, temperature sensor 13 is mounted on substrate 12. In the present exemplary embodiment, temperature sensor 13 includes a contact temperature sensor. Examples of the contact type temperature sensor include a thermistor, a thermocouple, and a resistance thermometer bulb.

Cooler 40 cools skin cooling unit 20. Since light irradiation hair removal device 1 includes cooler 40, the temperature of skin cooling unit 20 can be further lowered. Therefore, a skin cooling effect by skin cooling unit 20 can be further improved. Cooler 40 includes heat dissipation unit 41 and air blower 45.

Heat dissipation unit 41 is connected to skin cooling unit 20, and dissipates heat taken from skin cooling unit 20. Heat dissipation unit 41 includes connection unit 42, grip unit 43, and heat dissipation fin 44.

Connection unit 42 is a plate-shaped member. Substrate 12 is provided on a first surface, which is one surface of connection unit 42. Substrate 12 is smaller than connection unit 42, and is provided so as to be accommodated inside connection unit 42. Grip unit 43 is connected to outside of substrate 12 on the first surface of connection unit 42. Heat dissipation fin 44 is provided on a second surface, which is a surface opposite to the first surface of connection unit 42.

Grip unit 43 protrudes upward in up-down direction Z from the first surface of connection unit 42, and grips an entire peripheral edge of skin cooling unit 20. Therefore, light source 10 is surrounded by skin cooling unit 20, grip unit 43, and connection unit 42. The heat generated by light source 10 is dissipated via skin cooling unit 20 and heat dissipation unit 41 of cooler 40. Note that, while grip unit 43 grips the entire peripheral edge of skin cooling unit 20, grip unit 43 may be connected to at least a part of skin cooling unit 20.

Heat dissipation fin 44 is provided on the second surface of connection unit 42, which is a surface opposite to light source 10. Therefore, the heat of skin cooling unit 20 moves to heat dissipation fin 44 through grip unit 43 and connection unit 42. Heat dissipation fin 44 includes a plurality of fins, and has a large contact area with air, so that heat is easily dissipated.

Heat dissipation unit 41 preferably contains a material excellent in thermal conductivity. The value of the thermal conductivity of heat dissipation unit 41 may be larger than the value of the thermal conductivity of skin cooling unit 20. Specifically, heat dissipation unit 41 may contain a metal such as aluminum, iron, or copper. Grip unit 43, connection unit 42, and heat dissipation fin 44 may be made of the same material, or may be made of different materials.

Air blower 45 cools heat dissipation unit 41 by sending air to heat dissipation unit 41. Air blower 45 includes, for example, a fan, and when the fan rotates, an air flow is generated. Housing 5 is provided with a plurality of first opening portions 6 at positions facing air blower 45. Further, housing 5 is provided with a plurality of second opening portions 7 at positions facing heat dissipation fin 44. Therefore, when air blower 45 is driven, air taken in from outside of housing 5 through the plurality of first opening portions 6 is sent to heat dissipation fin 44. The heat of the air in contact with heat dissipation fin 44 is exchanged with the heat of heat dissipation fin 44, and heat dissipation fin 44 is cooled. The air heated in contact with heat dissipation fin 44 is discharged to outside of housing 5 through the plurality of second opening portions 7.

Note that, in the present exemplary embodiment, it has been described that light irradiation hair removal device 1 cools skin cooling unit 20 using air-cooled cooler 40, but skin cooling unit 20 may be cooled using a Peltier element or the like in addition to air-cooled cooler 40 or instead of air-cooled cooler 40. Light irradiation hair removal device 1 in a case of cooling skin cooling unit 20 using the Peltier element can cool skin cooling unit 20 more strongly.

Control unit 50 controls the ON state and the OFF state of light source 10. Further, control unit 50 controls a drive and stop of air blower 45. Control unit 50 has a computer system including a central processing unit (CPU), a read only memory (ROM), and a random access memory (RAM). Then, when the CPU executes programs stored in the ROM, the computer system functions as control unit 50. Here, the programs executed by the CPU are recorded in advance in the ROM of the computer system, but may be provided by being recorded in a non-transitory recording medium such as a memory card, or may be provided through a telecommunication line such as the Internet.

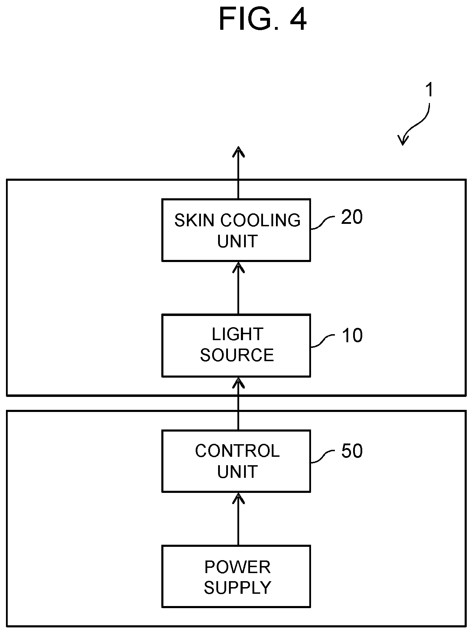

Fig. 4 is a block diagram of the major parts of light irradiation hair removal device 1 according to the present exemplary embodiment. As shown in the block diagram of Fig. 4, light irradiation hair removal device 1 includes a power supply, control unit 50, light source 10, and skin cooling unit 20, as a system that commands light irradiation. When the power supply is turned on by operating an operation switch (not illustrated) of light irradiation hair removal device 1, power is supplied to control unit 50, and a standby state of light irradiation is entered. During light irradiation, control unit 50 controls light source 10 to be on and off. Examples of the mode that starts the hair removal treatment include a mode in which the hair removal treatment is started when a user operates switches (not illustrated) provided in the body. In addition, it may be a mode in which a sensor that detects the skin is provided in light irradiation hair removal device 1, and light irradiation hair removal device 1 emits light when control unit 50 detects that the sensor has detected the skin.

Light irradiation hair removal device 1 of the present exemplary embodiment described above includes light source 10 and control unit 50. Light source 10 emits light having a wavelength of 400 nm to 1200 nm. Further, control unit 50 controls the on and off of light irradiation from light source 10. At single light irradiation from light source 10 during the hair removal treatment, control unit 50 controls light source 10 to repeat the ON state and the OFF state alternately and variably controls a time period of the ON state and a time period of the OFF state.

As a result, there is a rise in the temperature of the skin when light source 10 is in the ON state, but the temperature of the skin decreases when light source 10 is in the OFF state. Since such ON state and OFF state are alternately repeated, light irradiation hair removal device 1 can suppress a sudden rise in the skin temperature, and consequently, can suppress discomfort to the user. Further, assuming that total energy required for hair removal is 9 J/cm² to 50 J/cm², a sufficient hair removal effect can be obtained by setting total irradiation energy released in the ON state of light source 10 to be 9 J/cm² to 50 J/cm² in single light irradiation. In other words, light irradiation hair removal device 1 can suppress discomfort to the user regardless of individual properties of the user, while the sufficient total energy required for hair removal is provided.

On the other hand, in the conventional light irradiation hair removal device, there is no control of the ON state and the OFF state, and the irradiation energy required for hair removal is always provided in the ON state in single light irradiation, so that the skin temperature may suddenly rise, causing discomfort to the user.

In the present exemplary embodiment, "single light irradiation" means one continuous light irradiation performed on a certain skin region with irradiation energy to such an extent that a sufficient hair removal effect can be obtained. Here, "continuous" does not mean that the ON state of light source 10 is continuous, but means that a process in which the ON state and the OFF state of light source 10 are mixed is continuous.

In order to suppress the sudden rise in the temperature of the skin in single light irradiation, it is preferable to set low irradiation energy when starting light irradiation, and then gradually increase the irradiation energy. Then, since the user gets used to the irritation of heat generation by light irradiation, it is possible to suppress discomfort such as damage until the irradiation energy for obtaining a sufficient hair removal effect is obtained.

As described above, in order to gradually increase the irradiation energy, in the present exemplary embodiment, control unit 50 is preferably set to perform the control as below at single light irradiation from light source 10 during the hair removal treatment. In other words, control unit 50 preferably controls the ON state and the OFF state of light source 10 such that energy Eg(n) from light irradiation in the n^{th} ON state and energy Eg(n + 1) from light irradiation in the (n + 1)^{th} ON state satisfy the following conditions (1) and (2). Here, in the present exemplary embodiment, light source 10 is controlled such that the ON state and the OFF state are alternately repeated, but the n^{th} ON state and the (n + 1)^{th} ON state mean that the ON state of light source 10 is the first, the second,... the n^{th}, and the (n + 1)^{th} in single light irradiation. Note that, n is a natural number starting from 1. Condition (1): $Eg \left(n\right) \leq Eg \left(n+1\right)$ Condition (2): $Eg \left(n+1\right) = A \times Eg \left(n\right) A \geq 1$

The condition (1) defines that the energy Eg(n + 1) from light irradiation in the (n + 1)^{th} ON state is energy more than or equal to the energy Eg(n) from light irradiation in the n^{th} ON state. Further, the condition (2) defines that the energy Eg(n + 1) from light irradiation in the (n + 1)^{th} ON state is energy obtained by multiplying the energy Eg(n) from light irradiation in the n^{th} ON state by a constant A. In the condition (2), since the constant A is more than or equal to 1, the energy Eg(n + 1) from light irradiation in the (n + 1)^{th} ON state is A times more than or equal to the energy Eg(n) from light irradiation in the n^{th} ON state. In other words, in a case where the constant A is greater than 1, the energy of light irradiation gradually increases over time by satisfying the conditions (1) and (2).

As described above, in the present exemplary embodiment, as an example, the constant A is set to a number more than or equal to 1, and light irradiation is performed such that the energy Eg(n + 1) from light irradiation in the (n + 1)^{th} ON state becomes more than or equal to the energy Eg(n) from light irradiation in the n^{th} ON state. An adjustment of the energy from light irradiation can be performed by adjusting the light irradiation time, the number of light emitting elements (the number of LEDs) in light source 10, or the current, or a combination thereof. For example, energy adjustment by the light irradiation time can be performed by controlling the light irradiation time in a way of controlling light source 10 to be on and off. Further, the energy adjustment by the number of light emitting elements can be controlled by increasing or decreasing the number of light emitting elements to emit light. Further, the energy adjustment by the current can be performed by a current control circuit or the like. Among them, the energy adjustment by the light irradiation time can be realized only by controlling light source 10 to be on and off by control unit 50, and is most preferable in that no separate member is required. In other words, the energy adjustment by the light irradiation time can be realized at a low cost as compared with a case where the irradiation energy is controlled by a drive current of light source 10 or the number of light emitting elements.

As described above, in light irradiation hair removal device 1 of the present exemplary embodiment, control unit 50 preferably controls Eg(n) and Eg(n + 1) by adjusting the time period of the ON state of light source 10.

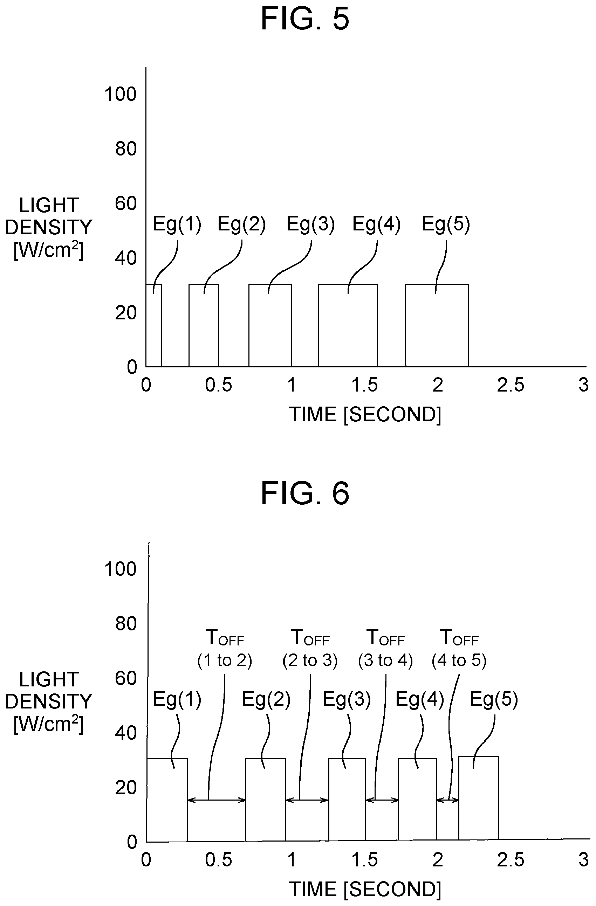

The graph of Fig. 5 shows one example of a relationship between elapsed time and light density during light irradiation of light irradiation hair removal device 1. More specifically, Fig. 5 illustrates a state in which the time period of the ON state of light source 10 (with a constant light density) is gradually increased in sequence from the first, and the energy from light irradiation (Eg(1) to Eg(5)) is gradually increased over time. Note that, in Fig. 5, rectangular areas of Eg(1) to Eg(5) indicate a magnitude of energy. Then, the total area may be sufficient total energy required for hair removal. With such a configuration, light irradiation hair removal device 1 can suppress discomfort to the user while applying the total energy required for hair removal to the skin.

**In** Fig. 5, a period between Eg(n) and Eg(n + 1) is the time period during which light irradiation is turned off. In Fig. 5, all the time periods during which light irradiation is turned off are equal, but the time periods during which light irradiation is in the OFF state do not necessarily have to be equal as long as the conditions (1) and (2) are satisfied.

In Fig. 5, the time period during which light irradiation is turned off can be, for example, 0.1 seconds to 1.0 second. Further, each of the energy Eg(1) to Eg(5) can be set to gradually increase, for example, between 0.1 seconds and 1.0 second.

In the condition (2), it is preferable to set 1 ≤ A ≤ 2. By setting the constant A to be less than or equal to 2, the skin surface temperature does not become too high, and occurrence of pain can be suppressed.

Further, the constant A may be provided with switches such that the user can change it in a range of 1 ≤ A ≤ 2. For example, a case where the constant A is 1 may be considered as a "weak" mode, a case where the constant A is 1.5 may be considered as a "medium" mode, and a case where the constant A is 2 may be considered as a "strong" mode.

The above preferred embodiment can also be expressed as follows. In other words, the light irradiation hair removal device of the preferred embodiment includes a plurality of LEDs having a wavelength peak at 400 nm to 1200 nm and a control unit that controls light emission of the LEDs. Then, the control unit is configured to repeatedly turn on and off the LEDs to control the radiation energy during LED-ON. The irradiation energy consists of a previous stage irradiation energy Eg(n) and a subsequent stage irradiation energy Eg(n + 1), and the previous stage irradiation energy Eg(n) ≤ the subsequent stage irradiation energy Eg(n + 1), and Eg(n + 1) = A × Eg(n) (n > 0 and 1 ≤ A). Here, the LEDs correspond to light source 10 in Figs. 1 to 4, and the control unit corresponds to control unit 50 in Figs. 2 and 4. Further, the effect of the light irradiation hair removal device of this embodiment is the same as the effect of light irradiation hair removal device 1 described above.

Note that, in the present exemplary embodiment, control unit 50 may be set to perform a control as follows at single light irradiation from light source 10 during the hair removal treatment. In other words, control unit 50 controls the energy Eg(n) from light irradiation in the n^{th} ON state and the energy Eg(n + 1) from light irradiation in the (n + 1)^{th} ON state so as to satisfy a condition (3). In addition, control unit 50 controls T_{OFF}(n to n + 1) and T_{OFF}(n + 1 to n + 2) so as to satisfy a condition (4). T_{OFF}(n to n + 1) is the time period of the OFF state of light source 10 between the n^{th} ON state and the (n + 1)^{th} ON state. T_{OFF} (n + 1 to n + 2) is the time period of the OFF state of light source 10 between the (n + 1)^{th} ON state and the (n + 2)^{th} ON state. Note that, n is a natural number starting from 1. Condition (3): $Eg \left(n\right) = Eg \left(n+1\right)$ Condition (4): ${T}_{OFF} \left(n to n+1\right) > {T}_{OFF} \left(n+1 to n+2\right)$

The condition (3) defines that the energy Eg(n) from light irradiation in the n^{th} ON state is equal to the energy Eg(n + 1) from light irradiation in the (n + 1)^{th} ON state. Further, the condition (4) defines that the time period T_{OFF} (n + 1 to n + 2) of the OFF state of light source 10 between the (n + 1)^{th} and the (n + 2)^{th} ON states is shorter than the time period T_{OFF} (n to n + 1) of the OFF state of light source 10 between the n^{th} and the (n + 1)^{th} ON states. In other words, when the conditions (3) and (4) are satisfied, the energy of light irradiation from the first to the (n + 1)^{th} ON state becomes constant, and the time period of the OFF state of light irradiation decreases over time. In other words, when the conditions (3) and (4) are satisfied, the energy of light irradiation gradually increases over time.

The graph of Fig. 6 shows another example of the relationship between elapsed time and light density during light irradiation of light irradiation hair removal device 1. More specifically, Fig. 6 illustrates a state in which all the time periods of the ON state of light source 10 (with a constant light density) is constant in the energy Eg(1) to Eg(5) from light irradiation. Further, Fig. 6 illustrates a state in which the time period T_{OFF}(1 to 2) to T_{OFF}(4 to 5) of the OFF state of light source 10 between each is gradually decreased over time. As shown in Fig. 6, an average energy Eg irradiated during a single irradiation time period gradually increases as the time period of the OFF state gradually decreases. Further, similarly to Fig. 5, the rectangular areas of Eg(1) to Eg(5) indicate the magnitude of energy. Then, the total area may be sufficient total energy required for hair removal. With such a configuration, light irradiation hair removal device 1 can suppress discomfort to the user while applying the total energy required for hair removal to the skin.

In Fig. 6, the time period during which light irradiation is turned on can be, for example, 0.1 seconds to 1.0 second. Further, each of the time period T_{OFF}(1 to 2) to T_{OFF}(4 to 5) during which light irradiation is turned off can be set to gradually decrease between 0.1 seconds and 1.0 second, for example.

In Fig. 6, in a case where the energy Eg(1) from the first light irradiation is large and cause discomfort to the user, the energy may be reduced by shortening the light irradiation time or the like for only Eg(1).

Light irradiation hair removal device 1 of the present exemplary embodiment preferably further includes a sensor that measures the skin surface temperature, and control unit 50 preferably controls the light irradiation time based on the skin surface temperature measured by the sensor. According to such a configuration, when the skin surface temperature is high, light irradiation hair removal device 1 can suppress the discomfort to the user by shortening the light irradiation time or pausing the light irradiation.

Note that, since the above-described exemplary embodiments are intended to illustrate the technique in the present disclosure, various changes, replacements, additions, omissions, and the like can be made within the scope of claims.

### INDUSTRIAL APPLICABILITY

The present disclosure is applicable to, for example, a hair removal device for home use and a hair removal device for business use.

### REFERENCE MARKS IN THE DRAWINGS

1: light irradiation hair removal device
5: housing
6: first opening portion
7: second opening portion
10: light source
12: substrate
13: temperature sensor
20: skin cooling unit
40: cooler
41: heat dissipation unit
42: connection unit
43: grip unit
44: heat dissipation fin
45: air blower
50: control unit

## Claims

1. A light irradiation hair removal device comprising:
a light source (10) adapted to emit light having a wavelength of 400 nm to 1200 nm; and
a control unit (50) adapted to control turning on and off of light irradiation from the light source (10); wherein
at single light irradiation from the light source (10) during a hair removal treatment, the control unit (50) is configured to (i) control the light source (10) to repeat an ON state and an OFF state alternately and (ii) variably a time period of the ON state and a time period of the OFF state; wherein
the control unit (50) is adapted to perform a control such that
energy Eg(n) from light irradiation in an n^{th} ON state and energy Eg(n + 1) from light irradiation in an (n + 1)^{th} ON state satisfy the following condition (3);
a time period T_{OFF}(n to n + 1) of the OFF state of the light source between the n^{th} ON state and the (n + 1)^{th} ON state and a time period T_{OFF}(n + 1 to n + 2) of the OFF state of the light source between the (n + 1)^{th} ON state and the (n + 2)^{th} ON state satisfy the following condition (4); and
time periods of the ON states are constant: condition (3): $Eg \left(n\right) = Eg \left(n+1\right)$ condition (4): ${T}_{OFFUS} \left(n+1 to n+2\right) > {T}_{AUS} \left(n to n+1\right) ;$ wherein
n is a natural number starting from 1.

## Patentansprüche

1. Vorrichtung für Entfernung von Haaren mittels Lichtbestrahlung, die umfasst:
eine Lichtquelle (10), die so eingerichtet ist, dass sie Licht mit einer Wellenlänge von 400 nm bis 1200 nm emittiert; sowie
eine Steuerungseinheit (50), die so eingerichtet ist, dass sie An- und Abschalten von Lichtbestrahlung durch die Lichtquelle (10) steuert; wobei
die Steuerungseinheit (50) so ausgeführt ist, dass sie bei einzelner Lichtbestrahlung durch die Lichtquelle (10) bei Haarentfernungs-Behandlung
(i) die Lichtquelle (10) so steuert, dass ein AN- und ein AUS-Status abwechselnd wiederholt werden, und
(ii) eine Zeitdauer des AN-Status und eine Zeitdauer des AUS-STATUS variabel steuert; wobei
die Steuerungseinheit (50) so eingerichtet ist, dass sie eine Steuerung so durchführt,
dass Energie Eg(n) von Lichtbestrahlung in einem n^{ten} AN-Status und Energie Eg(n + 1) von Lichtbestrahlung in einem (n + 1)^{ten} AN-Status die folgende Bedingung (3) erfüllen;
eine Zeitdauer T_{AUS} (n bis n + 1) des AUS-Status der Lichtquelle zwischen dem n^{ten} AN-Status und dem (n + 1)^{ten} AN-Status und eine Zeitdauer T_{AUS} (n + 1 bis n + 2) des AUS-Status der Lichtquelle zwischen dem (n + 1)^{ten} AN-Status
und dem (n + 2)^{ten} AN-Status die folgende Bedingung (4) erfüllen; und
Zeitdauern der AN-Status konstant sind: Bedingung (3): $Eg \left(n\right) = Eg \left(n+1\right)$ Bedingung (4): ${T}_{AUS} \left(n bis n+1\right) > {T}_{AUS} \left(n+1 bis n+2\right) ;$ wobei
N eine natürliche Zahl, beginnend mit 1, ist.

## Revendications

1. Dispositif d'épilation par irradiation lumineuse comprenant :
une source lumineuse (10) conçue pour émettre de la lumière ayant une longueur d'onde de 400 nm à 1 200 nm ; et
un module de commande (50) conçu pour commander l'activation et la désactivation d'une irradiation lumineuse par la source lumineuse (10) ; dans lequel
lors d'une irradiation lumineuse unique par la source lumineuse (10) pendant un traitement d'épilation, le module de commande (50) est configuré pour (i) commander la source lumineuse (10) pour qu'elle alterne répétitivement entre un état activé (ON) et un état désactivé (OFF) et (ii) commander de manière variable une durée de l'état ON et une durée de l'état OFF ;
dans lequel
le module de commande (50) est conçu pour mettre en œuvre une commande telle que
une énergie Eg(n) d'une irradiation lumineuse dans un n^{ième} état ON et une énergie Eg(n + 1) d'une irradiation lumineuse dans un (n + 1)^{ième} état ON satisfont à la condition (3) suivante ;
une durée T_{OFF}(de n à n + 1) de l'état OFF de la source lumineuse entre le n^{ième} état ON et le (n + 1)^{ième} état ON et une durée T_{OFF}(de n + 1 à n + 2) de l'état OFF de la source lumineuse entre le (n + 1)^{ième} état ON et le (n + 2)^{ième} état ON satisfont à la condition (4) suivante ; et
les durées des états ON sont constantes : condition (3): $Eg \left(n\right) = Eg \left(n+1\right)$ condition (4): ${T}_{OFF} \left(de n à n+1\right) > {T}_{OFF} \left(de n+1 à n+2\right) ;$ dans lequel
n est un entier naturel à partir de 1.
